Europäisches Patentamt

European Patent Office

Office européen des brevets

(19)

(11) **EP 0 753 293 B1**

(12) **EUROPÄISCHE PATENTSCHRIFT**

(45) Veröffentlichungstag und Bekanntmachung des Hinweises auf die Patenterteilung:
**28.05.2003 Patentblatt 2003/22**

(51) Int Cl.⁷: **A61M 15/00**

(21) Anmeldenummer: **96101748.0**

(22) Anmeldetag: **07.02.1996**

(54) **Vorrichtung zur Verabreichung von Substanzen, insbesondere Inhalationspräparate**

A device for administration of substances, especially of preparations for inhalation

Dispositif permettant d'administrer des substances, notamment des préparations pour inhalations

(84) Benannte Vertragsstaaten:
**CH DE FR GB IT LI**

(30) Priorität: **13.07.1995 DE 19525546**

(43) Veröffentlichungstag der Anmeldung:
**15.01.1997 Patentblatt 1997/03**

(73) Patentinhaber:
• **Schott Glas**
**55122 Mainz (DE)**
Benannte Vertragsstaaten:
**CH LI DE FR IT**
• **CARL-ZEISS-STIFTUNG trading as Schott Glas**
**55122 Mainz (DE)**
Benannte Vertragsstaaten:
**GB**

(72) Erfinder:
• **Reinhard, Michael, Dr.**
**D-55270 Ober-Olm (DE)**
• **Bouffleur, Ralf**
**D-55543 Bad Kreuznach (DE)**
• **Spallek, Michael, Dr.**
**D-55218 Ingelheim (DE)**
• **Geiger, Andreas**
**D-55286 Wörrstadt (DE)**

(56) Entgegenhaltungen:
WO-A-89/06793     WO-A-92/09323
DE-U- 29 511 334     US-A- 3 184 115
US-A- 3 432 072     US-A- 3 968 871

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft eine Vorrichtung zur Verabreichung von Substanzen, insbesondere Inhalationspräparate (Aerosole), mit Überdruck, umfassend einen Druckbehälter aus Glas, der wenigstens einen Vorrat einer abzugebenden Substanz und wenigstens eines Treibmittels enthält, und der eine Austrittsöffnung aufweist, auf der ein Abgabeorgan befestigt ist, einen Berst- und Splitterschutz aus Kunststoff, gebildet durch ein selbsttragendes Gehäuse das den Druckbehälter umgibt und das Abgabeorgan mit Ausnahme der Austrittsöffnung völlig umgreift, sowie Druckaustrittsöffnungen im Gehäuse,
einen Abgabeknopf, welcher mit dem Abgabeorgan so zusammenwirkt, dass bei Pressen beider Teile gegeneinander das Abgabeorgan betätigt wird, und eine bestimmte Menge der wenigstens einen abzugebenden Substanz die Austrittsöffnung durchtritt und in Form wenigstens eines Strahls aus einer Sprühöffnung im Angabekopf ausgestoßen wird.

Der Druckbehälter der eingangs beschriebenen Vorrichtung besitzt die Gestalt eines Fläschchens. Das Aufnahmevolumen des Fläschchens kann 5 - 50 ml betragen. Am oberen Ende des Fläschchens befindet sich die Austrittsöffnung. Diese wird in der Regel durch Einpressen und/oder Aufbördeln eines Abgabeorgans auf den Druckbehälter, beispielsweise eines Dosierventils mit einer nach oben ragenden meist zylindrischen Kanüle, vielfach unter Verwendung einer elastischen Dichtung zwischen Kopf und Dosierventil, verschlossen.

Der Druckbehälter wird in der Weise befüllt, dass die abzugebende(n) Substanz(en) vorab eingebracht oder gemeinsam mit dem(n) Treibmittel(n) durch das Dosierventil eingepresst werden.

Zur Vorbereitung der Verabreichung wird der Druckbehälter in ein zylindrisches Teil mit einem Abgabekopf eingeschoben, wobei die zylindrische Kanüle des Dosierventils formschlüssig in eine ebenfalls zylindrische mit einem Kanal verbundene, innen hohle Aufnahme des Abgabekopfes geführt sein kann. Der Kanal mündet in der Regel in eine in der Stirnseite des Abgabekopfes als Sprühkopf ausgebildete Öffnung.

Der Abgabekopf kann ein Mund- oder Nasenstück aufweisen, welches in einigen Fällen mit Hilfe einer aufsetzbaren Kappe gegen Verschmutzung geschützt werden kann.

Bei handelsüblichen Vorrichtungen ragt der Druckbehälter unten aus dem zylindrischen Teil mit Abgabekopf heraus.

Das Dosierventil kann dadurch aktiviert werden, dass gleichzeitig von oben mit dem Zeigefinger auf den Abgabekopf und von unten mit dem Daumen gegen den Druckbehälter gedrückt wird. In der Folge löst das Dosierventil aus und gibt über den Sprühkopf eine definierte Menge an Wirkstoff(en) frei.

Aus der US-A-3,184,115 ist eine Vorrichtung zur Verabreichung von Aerosolen mit Überdruck bekannt, welche aus einer unbeschichteten Glasflasche besteht, welche fest in einem Schutzgehäuse aus Kunststoff eingefasst ist, sowie eine Vorrichtung zum Dosieren und Versprühen des Aerosols. Die Schutzumhüllung aus Kunststoff ist stoßfest und soll eine Zerstörung der Glasflasche durch einwirkende Kräfte von außen verhindern. Weiterhin soll sie fest genug sein, um dem, bei einer Beschädigung der Glasflasche freiwerdenden Innendruck zu widerstehen und ein auseinander fliegen der Teile zu verhindern. Weiterhin sind in dem Bodenbereich des Schutzgehäuses aus Kunststoff kleine Öffnungen die die Form eines Schlitzes haben können angebracht, welche zum einen der Füllstandkontrolle der im Glas befindlichen Substanz dienen sollen, und welche zum anderen dem Entweichen des Druckes bei einem Bersten, der Flasche dienen sollen. Die Öffnungen sind hierbei so ausgelegt, dass bei einem Bersten der Glasflasche entstehende Glassplitter nicht durch die Öffnungen in dem Schutzgehäuses hindurchtreten können.

Nachteilig hierbei ist jedoch, dass auf Grund der örtlichen Begrenztheit dieser Öffnungen auf dem Bodenbereich sowie auf Grund der sehr geringen Spaltweite dieser Öffnungen eine genaue und vollständige Füllstandkontrolle der Substanz in der Flasche nur schwer möglich ist. Weiterhin ist bei einem Bruch oder Bersten der Flasche oberhalb ihres Bodenbereiches, was weitaus der häufigste Fall ist, eine Druckentspannung durch die im Bodenbereich befindlichen Öffnungen nicht gegeben, da das Schutzgehäuse aus Kunststoff die Flasche eng umschließt.

Des Weiteren sind handelsübliche Druckbehälter aus Glas bekannt, welche mit einer Beschichtung aus einem transparenten Kunststoff belegt sind. Diese Beschichtung soll als Splitterschutz beim Bersten des Behälters dienen und besteht daher aus einem flexiblen Weichkunststoff, z. B. PVC, mit hoher Dehnung. Die hohe Dehnung ist wichtig, da im Berstfall des Glasbehälters der Innendruck auf den Weichkunststoff einwirkt. Würde der Weichkunststoff nicht durch Volumenänderung dem Druck nachgeben können, ist mit einer plötzlichen Zerstörung des Weichkunststoffes durch Auflösung oder Spannungsrisskorrosion zu rechnen.

Eine derartige Vorrichtung hat den Vorteil, dass sie, da sowohl der Druckbehälter aus einem transparenten Material (Glas!) besteht als auch die Umhüllung, eine visuelle Ermittlung der Restmenge an abzugebender Substanz in dem Druckbehälter zulässt. Dies ist von besonderer Bedeutung bei der Verabreichung von Inhalationspräparaten, die vielfach den Charakter einer Notfallmedikation besitzen (z. B. Asthmapräparate). Hierbei besteht die Gefahr, dass ansonsten der Druckbehälter im Notfall leer ist.

Nachteilig an solch einer handelsüblichen Vorrichtung ist, dass der transparente Druckbehälter aus Glas trotz Beschichtung mit einem Kunststoff im Explosionsfall, z. B. durch unsachgemäße Handhabung, nicht sicher genug ist, da die Beschichtung speziell im Bereich des Ventils nicht verhindern kann, dass Teile des Glasbehälters geschossartig in die Umgebung geschleudert werden.

Es ist des Weiteren bekannt, Druckbehälter aus metallischen Werkstoffen einzusetzen, Der Berst- und Splitterschutz wird bei solchen Druckbehältern durch die diesbezüglich vorteilhaften Eigenschaften des ausgewählten Materials (hohe Zähigkeit, hohe Festigkeit) bewirkt.

Nachteilig an solch einer Vorrichtung ist jedoch, dass der nicht transparente Druckbehälter keine visuelle Ermittlung der in ihm noch vorhandenen Restmenge an abzugebender Substanz zulässt, was wiederum die oben beschriebenen Nachteile mit sich bringt.

Aufgabe der Erfindung ist, einen Druckbehälter der eingangs beschriebenen Art so weiterzubilden, dass der Vorteil der visuellen Ermittlung der Restmenge an abzugebender Substanz in dem Druckbehälter erhalten bleibt, andererseits aber der Berst- und Splitterschutz gegenüber bekannten Behältern beträchtlich erhöht wird.

Diese Aufgabe ist mit einer Vorrichtung und allen Merkmalen des Anspruchs 1 gelöst.

Bei der erfindungsgemäßen Vorrichtung kann das Gehäuse Druckaustrittsöffnungen aufweisen. Im Berstfall können die abzugebene Substanz und/oder das Treibmittel durch diese Druckaustrittsöffnungen entweichen. Dadurch das der Druckbehälterinhalt aus dem Gehäuse austreten kann, ist weiterhin die Gefahr eines chemischen Angriffs durch die Inhaltsstoffe auf das Gehäusematerial (Spannungsrückkorrosion) und damit die Explosionsgefahr weiter vermindert.

Dadurch das dass Gehäuse erfindungsgemäß in seiner Innenwandung ein System von Kanälen, d. h. Rillen aufweist, kann der Behälterinhalt, d. h. die abzugebene Substanz und/oder das Treibmittel schnellstmöglich durch die Kanäle zu den Druckaustrittsöffnungen geführt werden. In diesem Fall kann das Gehäuse unmittelbar an der Wandung des Druckbehälters anliegen, ohne das sich beim Bersten des Druckbehälters an irgendeiner Stelle in dem Gehäuse ein Druck aufbaut. Das Kanalsystem kann beispielsweise spiralförmig oder in der Form eines quadratischen Musters auf der Innenwandung des Gehäuses ausgeformt sein.

Das Gehäuse und der Druckbehälter sind nicht flächig fest miteinander verbunden. Dies hat gegenüber der Verwendung einer Kunststoffummantelung den Vorteil, dass Risse, die im Kunststoff entstehen, nicht wie im Fall der bekannten Vorrichtung, in die Glaswandung des Druckbehälters hineinwandern und diesen zum Bersten bringen zu können. Hieraus resultiert ein erhöhter Berstschutz.

Die erfindungsgemäße Vorrichtung ist des Weiteren recyclingfreudig, da sich Glasbehälter und Kunststoffgehäuse leicht voneinander trennen lassen.

Das Kunststoffgehäuse ist gemäß der Erfindung wenigstens im unteren Bereich des Druckbehälters aus einem transparenten Kunststoff gefertigt. Der Benutzer kann somit jederzeit feststellen, ob der Vorrat an abzugebender Substanz zur Neige geht und entsprechende Vorkehrung treffen.

Aufgrund des erhöhten Berst- und Splitterschutzes können auch dünnwandigere Behälter, beispielsweise bei Verwendung der Vorrichtung als Applikator für Inhalationspräparate pharmazeutische Fläschchen aus Glasrohr mit einer im Vergleich zu bekannten Applikatoren mit Glas-Druckbehältem um bis zu 40 % verminderten Wanddicke zum Einsatz kommen. Dies führt zu einer Materialersparnis bei der Herstellung der Druckbehälter für erfindungsgemäße Applikatoren. Da sich der Druckbehälter bei der erfindungsgemäßen Lösungen in seinen Abmessungen gegenüber bekannten Applikatoren nicht ändern, können für die Befüllung herkömmliche Abfüllanlagen verwendet werden.

Ein weiterer Vorteil dieser Erfindung besteht darin, dass das Eindringen von Fremdkörpern zwischen Druckbehältern und Gehäuse deutlich erschwert wird. Damit reduziert sich, insbesondere bei den oben erwähnten Applikatoren die Gefahr, aus diesem Bereich, durch den während der Applikation Luft gesaugt wird, Fremdkörper zu inhalieren.

Ein Kunststoff, der zur Herstellung des Gehäuses gemäß der Erfindung geeignet ist, muss folgende Eigenschaften besitzen: Transparenz, hohe Schlagzähigkeit, hohe Bruchdehnung. Folgende Materialien werden bevorzugt: Polycarbonat, Polyethylenterephtalat, amorphes Polyamid.

Vorzugsweise wird das Gehäuse durch zwei Halbschalen, z. B. eine obere und eine untere, gebildet, die nach Einbringen des Druckbehälters miteinander verbunden werden. Dies hat zum einen den Vorteil, dass die untere Halbschale bereits direkt nach der Produktion des Glasbehälters mit ihm verbunden werden kann. Dadurch wird der bei Druckbelastung am stärksten belastete Bodenbereich des Druckbehälters vor die Festigkeit herabsetzenden Oberflächendefekten geschützt und die hohe Zugfestigkeit des Glases bewahrt; zum anderen kann durch die Ausführung in zwei Halbschalen das Abgabeorgan zuerst auf den Behälter aufgebracht werden und danach die Halbschalen montiert und verbunden werden, damit die Halbschalen einen formschlüssigen Sicherheitsbehälter um den Glasbehälter mit Abgabeorgan bilden können.

Bei der oben beschriebenen zweiteiligen Anführungsform kann die untere Halbschale aus einem transparenten, die obere Halbschale dagegen zur Markierung aus einem farbigen Kunststoff bestehen, beispielsweise bei Applikatoren für Inhalationspräparate zur Kennzeichnung der Verwendung der Druckbehälter für unterschiedliche Medikamente.

Die Halbschalen können durch mechanische, physikalische oder chemische Methoden miteinander verbunden werden. Die Verbindung erfolgt z. B. durch Ultraschall-Schweißen, Reib-Schweißen, Heizelement-Schweißen, Kleben oder mechanisches Verhaken durch speziell geformte Ränder der Halbschalen. Dieses bewirkt eine feste Verbindung der Halbschalen miteinander.

Eine lösbare Verbindung wird bevorzugt, da sie das getrennte Recycling von Glas und Kunststoff erleichtert.

In einer bevorzugten Ausführungsform werden die beiden Halbschalen mittels übereinanderschiebbarer Widerhaken miteinander verhakt. Dies hat den Vorteil, dass der apparative Aufwand für dieses Verbindungssystem sehr gering ist und somit die Herstellungskosten für eine erfindungsgemäße Vorrichtung gering gehalten werden. Es ist darauf zu achten, dass die Verbindung eine Mindestzugfestigkeit berechnet aus folgender Formel aufweist:

$$\sigma m = 2{,}0 \text{ MPa} \times (d_a - s)/(2 \times s)$$

$\sigma m =$     Mindestzugfestigkeit der Verbindung [MPa]
$d_a =$     Außendurchmesser des Hohlkörpers [mm]
$s =$     Wanddicke des Hohlkörpers [mm]

(Anmerkung: 2.0 MPa entsprechen einem Innendruck von 20 bar).

Nachfolgend wird die Erfindung anhand der Figuren näher erläutert:

Es zeigen:

Fig. 1     einen Druckbehälter aus Glas mit Dosierventil für einen Applikator für Inhalationspräparate, der gemäß der Erfindung vollständig von einem selbsttragenden Gehäuse umgeben ist;

Fig. 2     eine bevorzugte Ausführungsform eines erfindungsgemäßen Applikators und

Fig. 3     eine weitere bevorzugte Ausführungsform eines erfindungsgemäßen Applikators.

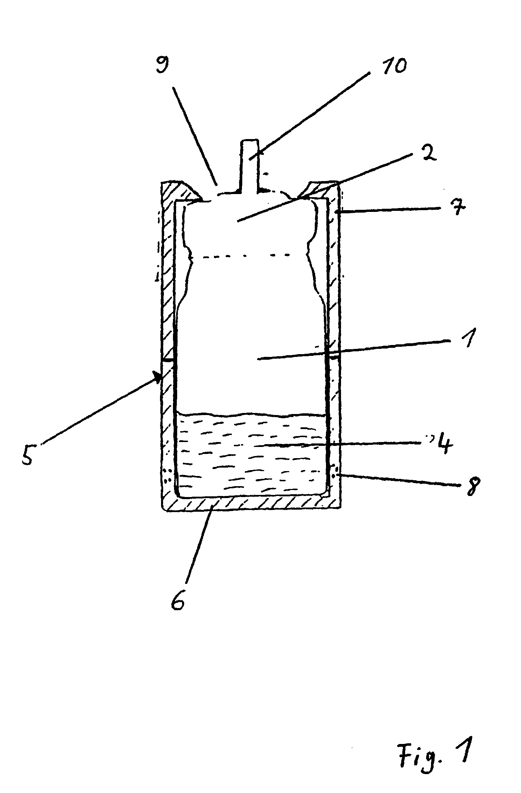

In Fig. 1 ist der Druckbehälter mit (1) bezeichnet. Der Druckbehälter besteht aus Glas, z. B. Borosilicatglas oder Kalk-Natron-Glas. Der Bodenbereich des Druckbehälters (1) ist vorzugsweise halbkugelförmig nach außen gewölbt, um auftretende Zugspannungen auf ein Minimum zu reduzieren. Der Druckbehälter (1) weist ein Dosierventil (2) auf, das durch Verkrimpen und/oder Aufbördeln im Bereich der Austrittsöffnung (3) befestigt ist. In dem Behälter (1) befindet sich das Aerosol (4), das unter Druck abgefüllt wird.

Der Druckbehälter (1) ist gemäß der Erfindung vollständig von einem Gehäuse (5) umgeben. Das Gehäuse (5) ist in der vorliegenden Ausführungsform zweiteilig ausgeführt. Es weist eine untere Halbschale (6) und eine obere Halbschale (7) auf. Man erkennt, dass das Gehäuse (5) im oberen Bereich des Druckbehälters (1) auch noch das Dosierventil (2) so weit umfasst, dass es nicht vom Behälter (1) weggeschleudert werden kann. Die untere Halbschale (6) ist ein zylindrisches Formteil aus transparentem Kunststoff, bevorzugt aus Polycarbonat oder Polyethylenterephtalat, das so bemaßt ist, dass es formschlüssig über den unteren Teil des Behälters geschoben werden kann. In der unteren Halbschale (6) sind Druckaustrittsöffnungen (8) in der Art und Weise integriert (z. B. durch seitliche Nuten oder durch Löcher im Bodenbereich), dass der bei Bruch des Druckbehälters plötzlich freigesetzte Behälterinhalt entweichen kann, ohne das Gehäuse (5) zu beschädigen. Gleichzeitig sorgt ein in der Figur nicht erkennbares System von Kanälen im Inneren der Halbschale (6) dafür, dass der unter Druck stehende Behälterinhalt (4) im Berstfall stehende Behälterinhalt (4) im Berstfall schnellstmöglich den Öffnungen (8) zugeführt und nach außen abgegeben wird. Die Öffnungen (8) sind so klein, dass sie keine Glassplitter durchlassen, die Personen verletzen könnten.

Die obere Halbschale (7) ist ebenfalls ein zylindrisches oder ein flaches rundes Formteil aus transparentem oder nicht transparentem Kunststoff, das vorzugsweise die gleichen Umfangsabmessungen wie die untere Halbschale (6) aufweist. Die Halbschale (7) ist so dimensioniert, dass sie formschlüssig über den oberen Teil des Druckbehälters (1), auf dem bereits das Dosierventil (2) aufgekrimpt und/oder aufgebördelt wurde, geschoben werden kann.

In der Halbschale (7) ist mindestens eine Öffnung (9) enthalten, die groß genug ist, um eine Durchführung des Ventilschaftes (10) oder anderer Ventilerhebungen zu ermöglichen.

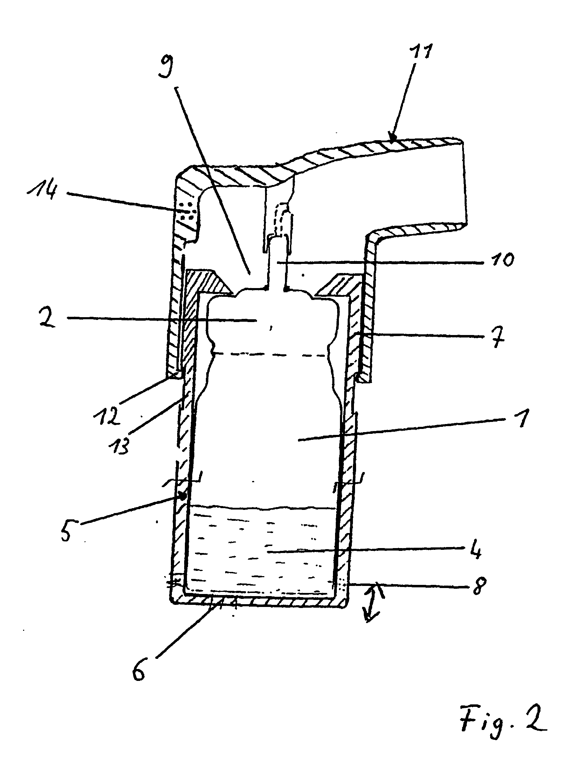

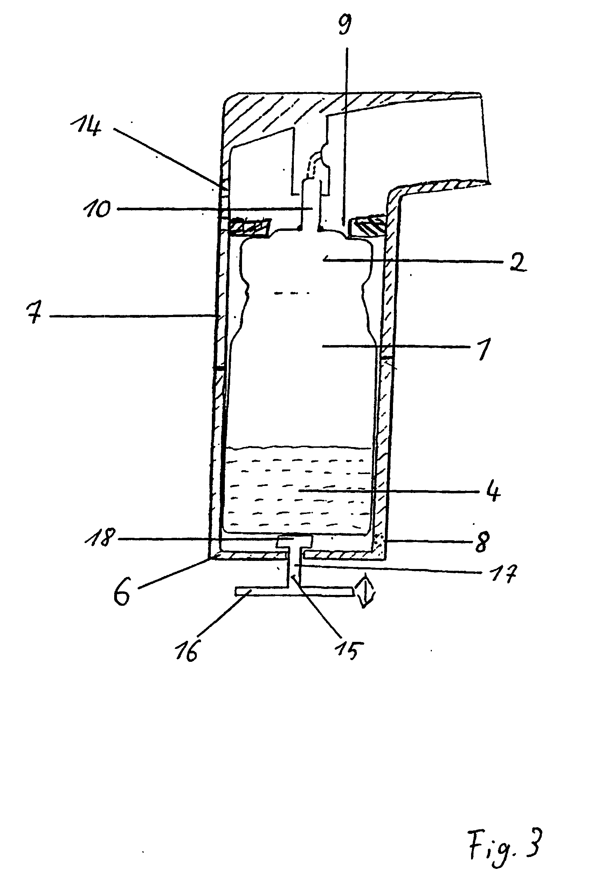

In den Fign. 2 und 3 sind bevorzugte Ausführungsformen eines erfindungsgemäßen Applikators dargestellt.

In beiden Figuren ist mit dem Bezugszeichen (11) der Abgabekopf mit der zylindrischen Aufnahme für den Ventilschaft (10) des Dosierventils bezeichnet, die Druckbehälter (1) mit Gehäuse (5) entsprechen in etwa der in Fig. 1 dargestellten Anordnung.

Bei beiden Ausführungsformen wird der Abgabekopf (11) am Gehäuse (5) gehaltert. Diese Art der Halterung hat insbesondere den Vorteil, dass der Ventilschaft (10) des Dosierventils (2) vor mechanischer Beanspruchung und Verstopfung durch Schmutzpartikel geschützt wird.

Vorteilhafterweise ist der Abgabekopf (11) so fest mit dem Gehäuse (5) verbunden, dass er sich bei normalem Gebrauch nicht von selbst lösen kann. Damit ist der Applikator jederzeit einsatzbereit. In den Figuren 2 und 3 sind ohne Beschränkung der Allgemeinheit zwei verschiedene Varianten für eine solche Verbindung aufgezeigt.

In Fig. 2 ist der Abgabekopf (11) von oben auf das Gehäuse (5) aufgesteckt und in dieser Position verankert. Hierzu weist der Abgabekopf (11) an seinem unteren Ende nach innen gerichtete Haken (12) auf, die in entsprechende Nuten (13) in der Gehäusewandung eingreifen. Die Nuten (13) besitzen eine Längsausdehnung, um eine Vertikalbewegung des Abgabekopfes (11) zur Betätigung des Dosierventils (2) zu ermöglichen. Das Dosierventil (2) wird durch Niederdrücken des Abgabekopfes (11) ausgelöst, wodurch über die Sprühöffnung eine definierte Menge an Wirkstoff freigegeben wird. Nach erfolgter Verabreichung wird der Abgabekopf (11) durch die Federkraft des Dosierventils (2) wieder in seine Ausgangsposition zurückgeführt.

Der Abgabekopf weist bei der in Fig. 2 gezeigten Ausführungsform auf der Rückseite Be- und Entlüftungsöffnungen (14) auf, die so ausgeführt sind, dass ein ungewolltes Verschließen durch den Anwender ausgeschlossen ist.

Bei der in Figur 3 dargestellten Ausführungsform ist der Abgabekopf (11) starr mit dem Gehäuse (5) verbunden, beispielsweise mittels Ultraschallschweißens. In diese Ausführungsform wird das Dosierventil (2) über eine von unten auf den Behälterboden einwirkende Aktivierungseinrichtung (15) ausgelöst.

Mit Hilfe der Aktivierungseinrichtung (15) kann der Druckbehälter (1) im Gehäuse (5) vertikal bewegt und dadurch das Dosierventil betätigt werden. Damit die Funktion des Systems gewährleistet wird, muss das Gehäuse (5) gegenüber dem in Fig. 2 gezeigten Ausführungsbeispiel um mindestens die Länge des Ventilbetätigungsweges verlängert werden.

Die Aktivierungseinrichtung (15) besteht aus einer Fingerauflage (16) und einem daran ansetzenden, in das Gehäuse (5) ragenden Steg (17), welcher am Ende eine Verdickung (18) aufweist, deren Durchmesser größer ist als der Durchmesser der Durchführung.

Im Inneren des Gehäuses (5) sitzt der Druckbehälter (1) mit dem Boden auf der Aktivierungseinrichtung (15). Zur Verabreichung des Medikaments wird die Fingerauflage (16) gegen das Gehäuse (5) gepresst. In der Folge drückt der Steg (18) auf den Boden des Druckbehälters (1), welcher sich so nach oben gegen den Federdruck des Dosierventils (2) verschiebt, so dass die zylindrische Kanüle (10) des Dosierventils (2) gegen die zylindrische Aufnahme des Abgabekopfes (11) drückt und das Dosierventil (2) auslöst.

**Patentansprüche**

1. Vorrichtung zur Verabreichung von Substanzen, insbesondere Inhalationspräparate (Aerosole), mit Überdruck, umfassend einen Druckbehälter (1) aus Glas, der wenigstens einen Vorrat einer abzugebenden Substanz und wenigstens eines Treibmittels enthält,
und der eine Austrittsöffnung (3) aufweist, auf der ein Abgabeorgan (2) befestigt ist,
einen Berst- und Splitterschutz aus Kunststoff (5), gebildet durch ein selbsttragendes Gehäuse (5) das den Druckbehälter (1) umgibt und das Abgabeorgan (2) mit Ausnahme der Austrittsöffnung (3) völlig umgreift, sowie Druckaustrittsöffnungen (8) im Gehäuse (5),
einen Abgabekopf (11), welcher mit dem Abgabeorgan (2) so zusammenwirkt, dass bei Pressen beider Teile gegeneinander das Abgabeorgan (2) betätigt wird, und eine bestimmte Menge der wenigstens einen abzugebenden Substanz die Austrittsöffnung (3) durchtritt und in Form wenigstens eines Strahls aus einer Sprühöffnung im Abgabekopf (11) ausgestoßen wird,
**dadurch gekennzeichnet,**
**dass** der Berst- und Splitterschutz durch ein Gehäuse (5) gebildet wird, welches auf seiner Innenwandung mit einem Kanalsystem versehen ist, das so ausgebildet ist, dass im Berstfall des Druckbehälters (1) die abzugebende Substanz und/oder das Treibmittel durch die Kanäle unmittelbar zu den Druckaustrittsöffnungen (8) geführt werden und das Gehäuse (5) wenigstens in seinem unteren Bereich aus einem transparenten Kunststoff besteht.

2. Vorrichtung nach Anspruch 1,
**dadurch gekennzeichnet,**
**dass** das Kanalsystem aus spiralförmig oder der Form eines quadratischen Musters auf der Innenwandung des Gehäuses (5) angeordneten Kanälen besteht.

3. Vorrichtung nach Anspruch 1.
**dadurch gekennzeichnet,**
**dass** das Gehäuse (5) aus zwei miteinander verbindbaren Halbschalen (6, 7) besteht, wobei die untere Halbschale (6) aus einem transparenten Kunststoff besteht.

4. Vorrichtung nach Anspruch 3,
**dadurch gekennzeichnet,**
**dass** die obere Halbschale (7) zur Kennzeichnung bestimmter Medikamente aus einem farbigen Kunststoff besteht oder eine farbige Markierung aufweist.

5. Vorrichtung nach Anspruch 3 oder 4,
**dadurch gekennzeichnet,**
**dass** die beiden Halbschalen (6, 7) lösbar miteinander verbunden sind.

6. Vorrichtung nach Anspruch 5,
**dadurch gekennzeichnet,**
**dass** die beiden Halbschalen (6, 7) mittels übereinander schiebbarer Widerhaken (12) miteinander verhakt sind.

7. Vorrichtung nach wenigstens einem der Ansprüche 1 - 6,
**dadurch gekennzeichnet,**
**dass** der Abgabekopf (11) am Gehäuse (5) gehaltert ist.

**8.** Vorrichtung nach Anspruch 7,
**dadurch gekennzeichnet,**
**dass** der Abgabekopf (11) fest mit dem Gehäuse (5) verbunden ist, und der Druckbehälter (1) zur Betätigung des Abgabeorganes (2) in dem Gehäuse (5) in Längsrichtung bewegbar ist.

**9.** Vorrichtung nach Anspruch 8,
**dadurch gekennzeichnet,**
**dass** der Druckbehälter (1) mittels einer von unten auf den Behälterboden einwirkenden Aktivierungseinrichtung (15) in dem Gehäuse (5) bewegbar ist.

**10.** Vorrichtung nach Anspruch 7,
**dadurch gekennzeichnet,**
**dass** der Druckbehälter (1) fest in dem Gehäuse (5) positioniert ist, und der Abgabekopf (11) zur Betätigung des Abgabeorganes (2) in Längsrichtung beweglich an dem Gehäuse (5) gehaltert ist.

**11.** Vorrichtung nach Anspruch 10,
**dadurch gekennzeichnet, dass** das Gehäuse (5) zur Führung und Halterung des Abgabekopfes (11) an seiner Außenseite sich in Längsrichtung erstreckenden Nuten (13) aufweist, in die entsprechend nach innen weisende Haken (12) am unteren Ende des Abgabekopfes (11) eingreifen.

## Claims

**1.** Device for administration of substances, in particular inhalation preparations (aerosols), under excess pressure, comprising a pressurized container (1) made of glass which has at least one supply of a substance to be dispensed and of at least one propellant, and which has an outlet opening (3) on which a dispensing member (2) is secured,
a means (5) made of plastic for protecting against bursting and splintering, formed by a self-supporting housing (5) which surrounds the pressurized container (1) and completely encloses the dispensing member (2) except for the outlet opening (3), and pressure outlet openings (8) in the housing (5),
a dispensing head which cooperates with the dispensing member (2) in such a way that, when the two parts are pressed against one another, the dispensing member (2) is actuated, and a specific amount of the at least one substance to be dispensed passes through the outlet opening (3) and is ejected in the form of at least one jet from a spray opening in the dispensing head (11),
**characterized in that**
the means protecting against bursting and splintering is formed by a housing (5) which on its inner wall is provided with a channel system designed in such a way that, if the pressurized container (1) bursts, the substance to be dispensed and/or the propellant are guided through the channels directly to the pressure outlet openings (8), and the housing (5) at least in its lower area consists of a transparent plastic.

**2.** Device according to Claim 1,
**characterized in that**
the channel system consists of channels arranged in a spiral formation or in the form of a square pattern on the inner wall of the housing (5).

**3.** Device according to Claim 1,
**characterized in that**
the housing (5) consists of two half-shells (6, 7) which can be connected to one another, the lower half-shell (6) consisting of a transparent plastic.

**4.** Device according to Claim 3,
**characterized in that**
in order to identify specific medicaments, the upper half-shell (7) consists of a coloured plastic or has a coloured marking.

**5.** Device according to Claim 3 or 4,
**characterized in that**
the two half-shells (6, 7) are connected to one another in a releasable manner.

**6.** Device according to Claim 5,
**characterized in that**
the two half-shells (6, 7) are hooked onto one another by means of mating hooks (12) which can be pushed over one another.

**7.** Device according to at least one of Claims 1-6,
**characterized in that**
the dispensing head (11) is retained on the housing (5).

**8.** Device according to Claim 7,
**characterized in that**
the dispensing head (11) is connected securely to the housing (5), and the pressurized container (1) is movable in the longitudinal direction in the housing (5) in order to actuate the dispensing member (2).

**9.** Device according to Claim 8,
**characterized in that**
the pressurized container (1) can be moved in the housing (5) by means of an activating device (15) acting on the container bottom from underneath.

**10.** Device according to Claim 7,
**characterized in that**
the pressurized container (1) is in a fixed position in the housing (5), and, in order to actuate the dispensing member (2), the dispensing head (11) is retained on the housing (5) so as to be movable in the longitudinal direction.

11. Device according to Claim 10, **characterized in that**, in order to guide and hold the dispensing head (11) on its outside, the housing (5) has grooves (13) which extend in the longitudinal direction and into which correspondingly inwardly directed hooks (12) at the lower end of the dispensing head (11) engage.

**Revendications**

1. Dispositif pour l'administration de substances, en particulier de préparations d'inhalation (aérosols), avec surpression, comprenant un récipient sous pression (1) en verre qui contient au moins une réserve d'une substance à délivrer et au moins un agent propulseur, et qui présente une ouverture de sortie (3) sur laquelle est fixé un organe d'émission (2),

   qui présente une protection contre l'éclatement et la rupture, en matière synthétique (5), formée par un boîtier autoportant (5) qui entoure le récipient sous pression (1) et qui englobe complètement l'organe d'émission (2) à l'exception de l'ouverture de sortie (3), ainsi que des ouvertures (8) de sortie de pression dans le boîtier (5),

   une tête d'émission (11) qui coopère avec l'organe d'émission (2) de telle sorte que lorsque les deux parties sont repoussées l'une contre l'autre, l'organe d'émission (2) est actionné et une quantité définie d'au moins une substance à délivrer traverse l'ouverture de sortie (3) et est expulsée sous la forme d'au moins un jet par l'ouverture de pulvérisation ménagée dans la tête d'émission (11),

   **caractérisé en ce que** la protection contre l'éclatement et la rupture est formée par un boîtier (5) qui est doté sur sa paroi intérieure d'un système de canaux qui est configuré de telle sorte qu'au cas où le récipient sous pression (1) éclate, la substance à délivrer et/ou l'agent propulseur sont guidés par les canaux directement vers les ouvertures (8) de sortie de pression et **en ce que** le boîtier (5) est au moins dans sa partie inférieure constitué d'une matière synthétique transparente.

2. Dispositif selon la revendication 1, **caractérisé en ce que** le système de canaux est constitué de canaux disposés en spirale ou sous la forme d'un motif en carré sur la paroi intérieure du boîtier (5).

3. Dispositif selon la revendication 1, **caractérisé en ce que** le boîtier (5) est constitué de deux demi-coquilles (6, 7) qui peuvent être reliées l'une à l'autre, la demi-coquille inférieure (6) étant constituée d'une matière synthétique transparente.

4. Dispositif selon la revendication 3, **caractérisé en ce que**, pour la caractérisation de certains médicaments, la demi-coquille supérieure (7) est constituée d'une matière synthétique colorée ou présente un repère coloré.

5. Dispositif selon la revendication 3 ou 4, **caractérisé en ce que** les deux demi-coquilles (6, 7) sont reliées l'une à l'autre de manière libérable.

6. Dispositif selon la revendication 5, **caractérisé en ce que** les deux demi-coquilles (6, 7) sont accrochées l'une à l'autre au moyen de barbes (12) pouvant coulisser l'un au-dessus de l'autre.

7. Dispositif selon au moins l'une des revendications 1 à 6, **caractérisé en ce que** la tête d'émission (11) est maintenue sur le boîtier (5).

8. Dispositif selon la revendication 7, **caractérisé en ce que** la tête d'émission (11) est reliée fixement au boîtier (5), le récipient sous pression (1) pouvant être déplacé dans le sens de la longueur dans le boîtier (5) pour l'actionnement de l'organe d'émission (2).

9. Dispositif selon la revendication 8, **caractérisé en ce que** le récipient sous pression (1) peut être déplacé dans le boîtier (5) au moyen d'un dispositif d'activation (15) qui agit depuis le bas sur le fond du récipient.

10. Dispositif selon la revendication 7, **caractérisé en ce que** le récipient sous pression (1) est positionné fixement dans le boîtier (5), la tête d'émission (11) étant maintenue à déplacement longitudinal dans le boîtier (5) pour l'actionnement de l'organe d'émission (2)

11. Dispositif selon la revendication 10, **caractérisé en ce que** pour le guidage et le maintien de la tête d'émission (11), le boîtier (5) présente sur son côté extérieur des rainures (13) qui s'étendent dans le sens de la longueur et dans lesquelles des crochets (12) correspondants, tournés vers l'intérieur, s'engagent à l'extrémité inférieure de la tête d'émission (11).

Fig. 1

Fig. 2

Fig. 3